# EUROPEAN PATENT APPLICATION

(11) **EP 1 876 447 A1**
(43) Date of publication of application: **09.01.2008**
(21) Application number: 06746055.0
(22) Date of filing: 27.04.2006
(51) Int. Cl.: G01N 33/574, C07K 16/18, C12Q 1/02

(54) **DIAGNOSTIC AGENT FOR TUMOR**

(30) Priority: 27.04.2005 JP 2005128990
(71) Applicant: Link Genomics, Inc., Chuo-ku Tokyo 103-0024 (JP)
(72) Inventor: NAKAO, Mitsuyoshi, Kumamoto-shi ,Kumamoto 8620908 (JP); ICHIMURA, Takaya, Kumamoto-shi, Kumamoto 8600079 (JP); ITO, Takaaki, Kumamoto-shi, Kumamoto 8620954 (JP)
(74) Representative: Baldock, Sharon Claire
(86) International application number: PCT/JP2006/309223
(87) International publication number: WO 2006/118338

(57) **Abstract**

The present invention provides a diagnostic agent and a diagnostic kit for tumor such as lung cancer, cervical cancer, gastric cancer, breast cancer or the like, which comprise an antibody against MCAF1 or MCAF2; and a diagnostic method using an antibody against MCAF1 or MCAF2.

SEQUENCE LISTING

<110> Link Genomics, Inc.

<120> Diagnostic agent for tumor

<130> G07-0115

<140> PCT/JP2006/309223
<141> 2006-04-27

<150> JP 2005-128990
<151> 2005-04-27

<160> 4

<210> 1
<211> 36
<212> DNA
<213> Artificial Sequence

<220>
<223> Description of Artificial Sequence: Synthetic DNA

<400> 1
cgccccggat ccatggacag tttagaagaa cctcag 36

<210> 2
<211> 42
<212> DNA
<213> Artificial Sequence

<220>
<223> Description of Artificial Sequence: Synthetic DNA

<400> 2
tttattctcg agaagctttg agtctttact ctgaatttgc tc 42

<210> 3
<211> 36
<212> DNA
<213> Artificial Sequence

<220>
<223> Description of Artificial Sequence: Synthetic DNA

<400> 3

cgggatccct cgagatggca agtccagata gaagta 36

<210> 4
<211> 38
<212> DNA
<213> Artificial Sequence
<220>
<223> Description of Artificial Sequence: Synthetic DNA

<400> 4
attggtaccc tcgagttacg taagattttc agaaaacc 38

SEQUENCE LISTING

<110> Link Genomics, Inc.

<120> Diagnostic agent for tumor

<130> P94957EP00

<140> EP 06746055.0
<141> 2006-04-27

<150> PCT/JP2006/309223
<151> 2006-04-27

<150> JP 2005-128990
<151> 2005-04-27

<160> 4

<210> 1
<211> 36
<212> DNA
<213> Artificial Sequence

<220>
<223> Description of Artificial Sequence: Synthetic DNA

<400> 1
cgccccggat ccatggacag tttagaagaa cctcag 36

<210> 2
<211> 42
<212> DNA
<213> Artificial Sequence

<220>
<223> Description of Artificial Sequence: Synthetic DNA

<400> 2
tttattctcg agaagctttg agtctttact ctgaatttgc tc 42

<210> 3
<211> 36
<212> DNA
<213> Artificial Sequence

<220>
<223> Description of Artificial Sequence: Synthetic DNA

<400> 3
cgggatccct cgagatggca agtccagata gaagta 36

<210> 4
<211> 38
<212> DNA
<213> Artificial Sequence
<220>
<223> Description of Artificial Sequence: Synthetic DNA

<400> 4
attggtaccc tcgagttacg taagattttc agaaaacc 38

## Description

### TECHNICAL FIELD

The present invention relates to an agent, a method and a kit for diagnosing tumor.

### BACKGROUND ART

Somatic cells, stem cells, immortalized cells in abnormal cases, and cancer cells variously differentiated in one living organism have different cell characters, but basically have the same genome. These cells have different cell characters because of difference in the gene expression patterns, which is caused by difference in the epigenetic state of the genome. In general, the epigenetic state of the genome is determined by DNA methylation and chromatin. It is known that such an epigenetic state is significantly changed in the process of generation and carcinogenesis of the cell.

Methylated DNA binging domain (MBD) protein specifically binds to methylated DNA and recruits deacetylated enzyme or methylated enzyme of histone molecules, heterochromatin protein HP1 or the like to form a transcription inactive chromatin. In order to cause DNA methylation and chromatin to cooperatively control gene expression in this manner, an MBD protein complex for linking the DNA methylation and the chromatin is necessary. However, agents bindable to the MBD protein have not been fully clarified.

In the meantime, it is very important in the clinical medicine and medical treatment to find and treat a tumorigenic lesion on an early stage. One useful technology to realize this is to detect a substance specific or relevant to tumor (hereinafter, referred to as a "tumor marker") in a bodily fluid, a tumor cell or a tumor tissue.

Conventionally known tumor markers include carcinoembryonic antigen (CEA), α-fetoprotein, tumor associated antigen (CA19-9, CA125, prostate specific antigen), enzyme (prostate acid phosphatase, LDH), oncogene product (neu/erbB2, N-myc), and hormone (heterotopic hormone such as hCG, ACTH, etc.). For detecting such tumor markers, radioimmunoassay (RIA) and enzyme-linked immunosorbent assay (ELISA) have been mainly used. Such conventional methods can detect cancers of specific tissues in a limited manner but cannot commonly detect various histologic types of tumorigenic lesions. Namely, the conventional tumor markers have a problem of not being efficient as an adjunctive diagnostic method for unknown histologic types of tumorigenic lesions.

One most common gynecologic cancer is cervical cancer. Diagnostic methods for cervical cancer include cytodiagnosis, biopsy and colposcopic examination. By cytodiagnosis, a cell scraped off from, or coming off from, a cancerous region is put on a glass plate, stained with a colorant and examined with a microscope. Since cervical cancer is often generated in the vicinity of the opening of uterus, a cell is scraped off from this region with a cotton swab or spatula for cytodiagnosis. By biopsy, a small tissue is taken from a region of cervix suspected to be cancerous to form a specimen, which is examined with a microscope. By colposcopic examination, the mucosa surface of cervix is enlarged with a magnifier-like device referred to as colposcope and examined in detail.

The progress of cervical cancer is classified into the following stages: stage 0: carcinoma in situ; stage I: limited to cervix; stage II: expanded beyond cervix but has not reached pelvic wall or lower 1/3 of vaginal wall; stage III: invasion has reached pelvic wall or lower 1/3 of vaginal wall; stage IV: expanded beyond lesser pelvis or invading mucosa of bladder or rectum. Before stage 0, dysplasia (mild, moderate, severe) occurs as a precancerous lesion. Namely, a cervical lesion progresses into carcinoma in situ and then invasive cancer after being in the state of mild dysplasia and severe dysplasia. However, all the cases of dysplasia are not developed into cancer. It is reported that 20% of severe dysplasia is developed into cancer, and 2.2% of mild dysplasia is developed into severe dysplasia and 0.3% of mild dysplasia is developed into cancer. By contrast, there is another report that dysplasia naturally disappears because the tissue is lost by biopsy and dysplasia is developed into cancer in almost all the cases where being watched with only cytodiagnosis and colposcopic examination. For cervical cancer, like cancers of other sites, early-stage finding and treatment is important to increase the curing ratio.

The results of cytodiagnosis are classified as follows: I: normal cells; II: inflammatory changes; IIIa: mild dysplasia; IIIb: severe dysplasia; IV: carcinoma in situ; and V: invasive cancer. The accuracy of cytodiagnosis is not high enough to allow the site to be determined as being cancerous with only cytodiagnosis.

### DISCLOSURE OF THE INVENTION

Under the above-described circumstances, a tumor marker useful for early-stage diagnosis of tumor is desired.

The present invention has an object of finding a tumor marker useful for early-stage diagnosis of tumor and providing an agent, a method and a kit for diagnosing tumor using the same.

The present inventors newly identified two human molecules (MCAF 1 and MCAF2) of MCAF (MBD1-containing chromatin-associated factor) as factors bindable to a transcription repression domain at the carboxylic terminus of human MBD1, by yeast 2 hybrid assay. These molecules were named MCAF/AM family since MCAF 1 is a human molecule homologous to a known mouse AM (ATFa-associated modulator). It has been found that MCAF1 is bound with SETBD1, which is a methylation enzyme of the ninth lysine of histone H3 (H3K9), to form an MBD1-MCAF1-SETBD1 complex, which is necessary for activating the methylated enzyme. MBD1-MCAF1-SETBD1 methylates H3K9 in a methylated DNA region to repress transcription and form heterochromatin. By contrast, in a non-methylated DNA region, it has been found that the MCAF molecules promote the transcription activation together with a transcriptional factor such as Sp1 or the like. Thus, the MCAF/AM family is positioned as a new epigenetics control factor.

The present inventors carried out western blotting or immunostaining using an MCAF polyclonal antibody to clarify that MCAF molecules are highly expressed in various types of human cultured cancer cell lines and cancer tissues. The expression of MCAF in cancer cell lines and cancer tissues was totally different from the low expression thereof in normal fibroblasts or normal tissues around cancer tissues. This suggests that MCAF molecules are relevant to cancer generation and can be tumor markers for detecting a wide range of cancer cells or cancer tissues.

A cancer cell is largely different from a normal cell in the epigenetic state of genome and heterochromatin. This suggests that the transcription control and chromatin formation by MCAF family molecules possibly play an important role.

For initial cancer screening, it is important that a tumor marker capable of detecting a wide range of histologic types of tumorigenic lesions with high sensitivity is provided, not a tumor marker for detecting a specific histologic type of cancer. MCAF protein used in the present invention is a tumor marker suitable for this purpose, and realizes tumor diagnosis by immunostaining performed on tumor cells or tumor tissues using a specific polyclonal antibody. For example, the stainability of MCAF1 is usable to distinguish normal cells which are not stained from tumor cells which are strongly stained.

The present inventors also performed immunostaining on samples derived from subjects suspected to have cervical cancer or cervical cancer patients using an anti-MCAF 1 antibody, and found that MCAF 1 is highly expressed also in precancerous lesion, initial invasive cancer, and lymph node metastasis of cervical cancer. Especially, the high expression of MCAF 1 in precancerous lesion and lymph node metastasis indicates that MCAF protein can be a tumor marker very useful for early-stage treatment and establishment of an accurate treatment principle of cervical cancer.

Accordingly, the present invention provides the following.
(1) A diagnostic agent for tumor, comprising an antibody against MCAF1 or MCAF2.
(2) A diagnostic agent for tumor, comprising F(ab')₂ fragment or Fab' fragment of an antibody against MCAF 1 or MCAF2.
(3) The diagnostic agent according to (1) or (2), wherein the tumor is lung cancer, cervical cancer, gastric cancer or breast cancer.
(4) The diagnostic agent according to (3), wherein the tumor is cervical cancer.
(5) A diagnostic method for tumor, comprising the steps of:
   putting a sample derived from a subject into contact with an antibody against MCAF1 or MCAF2; and
   detecting or measuring the MCAF1 or MCAF2 in the sample.
(6) The diagnostic method according to (5), wherein F(ab')₂ fragment or Fab' fragment of the antibody against the MCAF1 or MCAF2 is used as the antibody against the MCAF1 or MCAF2.
(7) The diagnostic method according to (5) or (6), which is performed in accordance with enzyme immunoassay or radio immunoassay.
(8) The diagnostic method according to any one of (5) through (7), wherein the tumor is lung cancer, cervical cancer, gastric cancer or breast cancer.
(9) The diagnostic method according to (8), wherein the tumor is cervical cancer.
(10) The diagnostic method according to any one of (5) through (9), wherein the sample is tissue, cell, serum, saliva or urine derived from the subject.
(11) A diagnostic kit for tumor, comprising an antibody against MCAF1 or MCAF2.
(12) A diagnostic kit for tumor, comprising F(ab')₂ fragment or Fab' fragment of an antibody against MCAF1 or MCAF2.
(13) The diagnostic kit according to (11) or (12), wherein the tumor is lung cancer, cervical cancer, gastric cancer or breast cancer.
(14) The diagnostic kit according to (13), wherein the tumor is cervical cancer.
(15) A diagnostic agent for precancerous lesion, comprising an antibody against MCAF1 or MCAF2.
(16) The diagnostic agent according to (15), wherein the precancerous lesion is precancerous lesion of cervix.
(17) A diagnostic kit for precancerous lesion, comprising an antibody against MCAF 1 or MCAF2.
(18) The diagnostic kit according to (17), wherein the precancerous lesion is precancerous lesion of cervix.
(19) A diagnostic method for precancerous lesion, comprising the steps of:
   putting a sample derived from a subject into contact with an antibody against MCAF 1 or MCAF2; and
   detecting or measuring the MCAF1 or MCAF2 in the sample.
(20) A diagnostic method for precancerous lesion of cervical cancer, comprising the steps of:
   putting a sample derived from cervix or a tissue in the vicinity thereof of a subject into contact with an antibody against MCAF1 or MCAF2; and
   detecting or measuring the MCAF1 or MCAF2 in the sample.
(21) A diagnostic agent for lymph node metastasis, comprising an antibody against MCAF1 or MCAF2.
(22) The diagnostic agent according to (21), wherein the lymph node metastasis is lymph node metastasis of cervical cancer.
(23) A diagnostic kit for lymph node metastasis, comprising an antibody against MCAF1 or MCAF2.
(24) The diagnostic kit according to (23), wherein the lymph node metastasis is lymph node metastasis of cervical cancer.
(25) A diagnostic method for lymph node metastasis, comprising the steps of:
   putting a sample derived from lymph node of a subject into contact with an antibody against MCAF 1 or MCAF2; and
   detecting or measuring the MCAF1 or MCAF2 in the sample.
(26) The diagnostic method according to (25), which is for diagnosing lymph node metastasis of cervical cancer.

Substantially no stained MCAF1 protein or MCAF2 protein is seen in normal cells or tissues, but especially MCAF 1 has been recognized to have significantly strong stainability in a wide range of histologic types of malignant tumors (lung cancer, uterine cancer, cervical cancer, etc.). Accordingly, it is considered that MCAF protein is useful as a novel tumor marker, and that immunostaining using a specific antibody against MCAF protein is usable for pathologically diagnosing various histologic types of tumorigenic lesions.

Immunostaining performed on a clinical sample (cytodiagnosis, biopsy, surgically excised tissue, etc.) using a polyclonal antibody against MCAF protein can identify a wide range of histologic types of tumor cells, identify tumor cells in primary lesion and invaded or metastatic lesion, and clarify the border between a normal tissue and a tumor tissue. Thus, the immunostaining is expected to greatly contribute to diagnosis, treatment and recuperation of tumorigenic lesion.

According to a method, an agent and a kit for diagnosing tumor according to the present invention which use an antibody against MCAF1 or MCAF2 protein, cervical cancer can be found on an early stage. For example, screening to find a group of precancerous lesions (severe dysplasia) before stage 0 which have a risk of developing into cervical cancer, or diagnosis on cervical cancer on an early stage (for example, stage I (limited to cervix) to stage II (initial invasive cancer)) are possible. Accordingly, the present invention is usable to prevent cervical cancer and improve the curing ratio by early-stage treatment.

The present invention is also usable for diagnosing lymph node metastasis of cervical cancer and has an advantage of allowing an accurate treatment principle to be established.

### BRIEF DESCRIPTION OF THE DRAWINGS

Fig. 1 shows optical micrographs presenting results of MCAF1 immunostaining in normal lung tissues and different histologic types of lung cancers.
Fig. 2 shows optical micrographs presenting results of MCAF1 immunostaining in cervical cancer, uterine body cancer (or endometrial cancer), gastric cancer and breast cancer.
Fig. 3 shows an optical micrograph presenting results of MCAF1 immunostaining in a sample derived from precancerous lesion (severe dysplasia) of a cervical cancer patient.
Fig. 4 shows an optical micrograph presenting results of MCAF1 immunostaining in a sample derived from early stage (initial invasive cancer) of a cervical cancer patient.
Fig. 5 shows an optical micrograph presenting results of MCAF1 immunostaining in a sample derived from lymph node metastasis of a cervical cancer patient.

### BEST MODE FOR CARRYING OUT THE INVENTION

Hereinafter, embodiments of the present invention will be described.

The present inventors found that MCAF1 and MCAF2 are each a useful tumor marker for diagnosing tumor. Namely, the present invention relates to a tumor diagnostic agent comprising an antibody against MCAF1 or MCAF2.

The amino acid sequences of MCAF1 and MCAF2 are known. For example, human MBD1-containing chromatin associated factor-1 (MCAF1, previously referred to as "MCAF") has been registered with Registration No. AF425650 in the NCBI database, and is also described in Mol. Cell. Biol. Vol. 23, pp. 2834-2843, 2003. Human MBD1-containing chromatin associated factor-2 (MCAF2) has been registered with Registration No. NM024997 in the NCBI database. Accordingly, those skilled in the art would easily obtain MCAF1 protein, MCAF2 protein and genes encoding the same. Herein, MCAF1 protein and MCAF2 protein will be occasionally referred to simply as "MCAF1" and "MCAF2", respectively.

### (Diagnostic agent)

The present invention provides a tumor diagnostic agent including an antibody against MCAF 1 or MCAF2.

As used herein, the term "anti-MCAF1 (or MCAF2) antibody" or "antibody against MCAF1 (or MCAF2)" refers to an antibody specifically bindable to MCAF1 (or MCAF2). As described later in more detail, an antibody used in the present invention may be produced by a conventional method including immunizing an animal and recovering serum (polyclonal) or spleen cell (for producing hybridoma by fusion with an appropriate cell).

As used herein, when an antibody is expressed as being "specifically bindable" to a protein or a fragment thereof, this means that the antibody is bindable to a specific amino acid sequence of the protein or a fragment thereof with substantially higher affinity than to other amino acid sequences. As used herein, the expression "substantially higher affinity" refers to affinity which is sufficiently high for the specific amino acid sequence to be detected as being distinguished from the other amino acid sequences by a desired measuring apparatus. Typically, the expression "substantially higher affinity" refers to binding affinity having a binding constant (Kₐ) of at least 10⁷M⁻¹, preferably at least 10⁸M⁻¹, more preferably 10⁹M⁻¹, still more preferably 10¹⁰M⁻¹, 10¹¹M⁻¹, 10¹²M⁻¹ or higher, for example, 10¹³M⁻¹ or higher at the maximum.

An antibody against MCAF1 or MCAF2 used in the present invention may be a polyclonal antibody or a monoclonal antibody, which can be prepared by a method known to those skilled in the art (see, for example, Shin Seikagaku Jikken Koza 1, Tanpakushitsu I (Sequel to Biochemistry Experiment Seminar 1, Protein I), pp. 389-406, Tokyo Kagaku Dojin Co., Ltd.). MCAF1 and MCAF2 proteins, the amino acid sequences of which are known as described above, can be produced by a usual protein expression technology based on the amino acid sequences. A partial peptide of MCAF 1 or MCAF2 can be produced by a usual peptide synthesis technology by selecting an appropriate partial sequence from the amino acid sequence of MCAF 1 or MCAF2.

A polyclonal antibody against MCAF 1 or MCAF2 is prepared by, for example, as follows. An appropriate amount of MCAF1 or MCAF2 protein or a partial peptide thereof is administered into an animal such as rabbit, guinea pig, mouse, chicken or the like. The MCAF1 or MCAF2 protein or a partial peptide thereof may be administered together with an adjuvant for promoting the antibody production (FIA or FCA). The administration is usually performed every several weeks. By performing immunization a plurality of times, the antibody titer can be increased. After the final immunization, blood is sampled from the immunized animal. Thus, antiserum is obtained. The antiserum is subjected to, for example, fractionation by ammonium sulfate precipitation or anionic chromatography or to affinity purification using protein A or immobilized antigen. As a result, a polyclonal antibody is prepared.

A monoclonal antibody against MCAF1 or MCAF2 is prepared by, for example, as follows. An animal is immunized with MCAF1 or MCAF2 protein or a partial peptide thereof as described above. After the final immunization, spleen or lymph node is sampled from the immunized animal. Antibody producing cells and myeloma cells included in the spleen or lymph node are fused together using polyethyleneglycol or the like to prepare hybridomas. An intended hybridoma is obtained by screening and cultured. From the resultant supernatant, a monoclonal antibody is prepared. The monoclonal antibody may be purified by, for example, fractionation by ammonium sulfate precipitation or anionic chromatography or affinity purification using protein A or immobilized antigen. For the purpose of the present invention, the prepared antibody may recognize any epitope of MCAF1 or MCAF2.

According to the present invention, a fragment of the above-described antibody may be used. Exemplary fragments of the antibody include F(ab')₂ fragment, Fab' fragment and the like. These fragments are assumed to exhibit substantially the same antigen specificity as that of the original antibody (such a fragment will be referred as a "functional fragment" herein). Functional fragments of the antibody include Fab, Fab', F(ab')₂, single chain antibody (scFv), disulfide stabilized V region fragment (dsFv) or CDR-containing peptide. Functional fragments of the antibody also include, for example, derivatives such as human type (or humanized) antibody (for example, CDR-transplanted complete human type antibody).

For the accuracy as a diagnostic agent, the antibody against MCAF1 or MCAF2 is preferably a human type antibody or a human antibody. A human type antibody, for example, a mouse-human chimera antibody may be prepared by isolating antibody gene from mouse cells which are for producing an antibody against MCAF 1 or MCAF2 protein, changing a H chain constant region thereof into a human IgE H chain constant region gene by recombination, and introducing the human IgE H chain constant region gene into mouse myeloma cells. A human antibody may be prepared by immunizing mouse, in which the immune system has been replaced by the human immune system, with MCAF 1 or MCAF2 protein.

As specifically described in the examples herein, a diagnostic agent according to the present invention including an antibody against MCAF1 or MCAF2 is suitable to diagnosis of any disease which is characterized by expression or high expression of MCAF1 or MCAF2 (for example, cervical cancer, uterine body cancer, lung cancer, breast cancer, gastric cancer or the like).

An antibody against MCAF1 or MCAF2 usable for a tumor diagnostic agent according to the present invention may be optionally labeled with a labeling substance for monitoring or the like (for example, radioisotope, fluorescent substance, etc.).

A pharmaceutical drug, in which an antibody against MCAF 1 or MCAF2 usable in the present invention is labeled with a labeling substance such as a radioisotope, a fluorescent substance or the like, may be produced based on a known technique.

A diagnostic agent according to the present invention may optionally include, in addition to an antibody against MCAF1 or MCAF2 as described above, an appropriate carrier, an appropriate excipient or the like in accordance with the normal technique. For example, a surfactant, an excipient, a colorant, a flavoring agent, a preservative, a stabilizer, a buffering agent, a suspending agent, an isotonizing agent, a binder, a disintegrator, a lubricant, a fluidity promoter, a sweetening agent or the like may be added. Usable additives are not limited to these, and other normally usable carriers may be added.

There is no specific limitation on the type of tumor which can be diagnosed by a diagnostic agent according to the present invention. All the benign and malignant tumors are diagnosable. Specific examples of the malignant tumor include, but are not limited to, malignant melanoma, malignant lymphoma, cancer of digestive organs, lung cancer, esophageal cancer, gastric cancer, colorectal cancer, rectal cancer, colon cancer, ureteral tumor, gallbladder cancer, bile duct cancer, biliary tract cancer, breast cancer, liver cancer, pancreatic cancer, testicle tumor, maxillary cancer, tongue cancer, lip cancer, oral cancer, pharyngeal cancer, laryngeal cancer, ovary cancer, uterine cancer, cervical cancer, uterine body cancer, prostate cancer, thyroid cancer, brain tumor, Kaposi's sarcoma, angioma, leukemia, genuine plethora, neuroblastoma, retinoblastoma, myeloma, bladder tumor, sarcoma, osteosarcoma, myosarcoma, skin cancer, basalioma, cancer of appendages of skin, metastatic skin cancer, skin melanoma, etc. Herein, the terms "cancer" and "tumor" are used interchangeably.

### (Diagnostic method)

The present invention also provides a tumor diagnostic method including putting a sample into contact with an antibody against MCAF1 or MCAF2 or the above-described diagnostic agent according to the present invention.

According to the present invention, the "sample" encompasses cell, tissue, serum, saliva, urine and the like obtained from a subject suspected of having tumor or a subject having tumor. Especially preferably, the "sample" is tissue or serum. The sample may be put into contact with the antibody by a method generally used in the art with no specific limitation. Diagnosis may be carried out by, for example, putting the sample into contact with the antibody, quantitatively detecting specific binding of MCAF 1 or MCAF2 which may be existent in the sample and the antibody using, for example, a secondary antibody labeled with a fluorescent substance, a luminescent substance, an enzyme or the like. The reaction for diagnosis may be carried out in a liquid phase such as a well or the like, or on a solid phase support having the antibody against MCAF1 or MCAF2 immobilized thereon. Such a method is usable for diagnosing diseases characterized by higher expression of MCAF1 or MCAF2 than, for example, normal cells, typically for dignosing cancer including lung cancer, cervical cancer, gastric cancer, breast cancer and the like. Usually, the level (or the amount) of MCAF1 or MCAF2 in a biological sample is evaluated based on the level (or the amount) of the complex of MCAF1 (or MCAF2) and the anti-MCAF1 (or MCAF2) antibody. The level of MCAF1 or MCAF2 in the biological sample is compared with the level of MCAF1 or MCAF2 in a control of normal human, and the existence of cancer is diagnosed based on the result (or the difference).

In this case, the measurement value of the above sample may be compared with a reference value obtained in advance using a normal sample without tumor or a sample found to have tumor, so that it is determined whether the measurement value is positive or not in terms of the existence of tumor. For diagnosis, a cutoff value may be set based on the measurement results of the amounts of MCAF1 or MCAF2 in sera of many tumor patients and healthy humans. Usually, a level of MCAF1 or MCAF2 higher than that of a normal human control indicates the existence of cancer. A MCAF1 or MCAF2 level which is more preferably at least twice, and most preferably about five times the level in the normal human control, is a positive result indicating that the subject has tumor. Alternatively, the existence per se of MCAF1 or MCAF2 in a biological sample (i.e., the amount of MCAF1 or MCAF2 detectable by available detection means) may indicate that the subject has tumor.

Specific exemplary methods for immunologically detecting MCAF1 or MCAF2 in a sample using an antibody against MCAF1 or MCAF2 include enzyme immunoassay (EIA) such as sandwich ELISA or the like, radio immunoassay (RIA) and the like, which are well-known to those skilled in the art. For example, the sandwich ELISA is performed as follows. Two types of antibodies recognizing different sites of antigen are prepared. One antibody is caused to adsorb to a plate. A sample is put into contact with the plate and reacted. The other antibody recognizing a different site of antigen is reacted. Also, an anti-immunoglobulin antibody labeled with an enzyme such as peroxidase, alkaline phosphatase or the like is reacted. After a substrate which can be colored by an enzyme is added to cause coloring reaction, the coloring strength is measured by a spectrometer. Thus, MCAF1 or MCAF2 in the sample may be detected or measured. By radio immunoassay, substantially the same procedure as enzyme immunoassay is performed but using an antibody labeled with a radioactive substance such as ¹²⁵I or the like instead of an enzyme, and the radiation is measured by a scintillation counter.

Any of the following measuring methods is usable for the present invention as well as the measuring methods described above as examples: western blotting, sandwich immunoassay, fluorescence immunoassay (FIA), time-resolved fluorescence immunoassay (TRFIA), enzyme immunoassay (EIA), luminescence immunoassay (LIA), electrochemical luminescence immunoassay (ECLIA), latex aggregation, immunoprecipitation assay, precipitin reaction, gel diffusion precipitin reaction, immunodiffusion assay, agglutin assay, complement fixation assay, immunoradiometric assay, fluoroimmunoassay, protein A immunoassay, etc.

A diagnostic method according to the present invention is usable for diagnosing whether or not the subject has tumor, and is also usable over a certain time period for confirming the effect of the treatment performed on the tumor.

As used herein, when the expression "cervix or tissues in the vicinity thereof' is used, "tissues in the vicinity thereof' refers to tissues around cervix, for example, tissues to which cervical cancer may possibly expand, such as parametrium, pelvic wall, mucosas of bladder and rectum, etc.

### (Diagnostic kit)

The present invention further provides a tumor diagnostic kit comprising an antibody against MCAF1 or MCAF2. A kit according to the present invention comprises at least an antibody against MCAF 1 or MCAF2 as described above, and may also include, for example, a reagent (for example, secondary antibody, coloring reagent, buffer solution, etc.) necessary for detecting MCAF1 or MCAF2 in the sample, or a user's manual provided by the manufacturer. The antibody against MCAF1 or MCAF2 may be included in the kit according to the present invention in the state of being bound to a solid phase support, for example, a polystyrene plate such as a microtiter plate or the like, glass or synthetic resin particles (beads), glass or synthetic resin spheres (balls), latex, magnetic particles, any of various types of membranes such as a nitrocellulose membrane, a synthetic resin test tube, a silica gel plate or the like.

Hereinafter, the present invention will be described by way of examples, but the present invention is not limited to these examples.

### Examples

### Example 1: Production of recombinant protein of MCAF 1 and a polyclonal antibody against MCAF 1

### (1) Synthesis of recombinant protein of MCAF1

cDNA corresponding to an amino group terminus region of human MCAF1 (amino acid number: 1-334) is amplified by PCR using the following synthetic oligonucleotide primers. As the template, a vector of pcDNA3-MCAF1 (Ichimura T, Watanabe S, Sakamoto Y, Aoto T, Fujita N, and Nakao M., Transcriptional repression and heterochromatin formation by MBD1 and MCAF/AM family proteins, J. Biol. Chem. 280: 13928-13935, 2005; and Fujita N, Watanabe S, Ichimura T, Ohkuma Y, Chiba T, Saya H, Nakao M., MCAF mediates MBD1-dependent transcriptional repression., Mol. Cell. Biol. 23: 2834-2843, 2003) is used. The amplification is performed by repeating 35 times the cycle of reaction at 94°C for 1 minute, 60°C for 1 minute, and 72°C for 2 minutes. As the primer sequences, the sense strand of 5'-CGCCCCGGATCCATGGACAGTTTAGAAGAACCTCAG-3' (SEQ ID NO: 1) and the anti-sense strand of 5'-TTTATTCTCGAGAAGCTTTGAGTCTTTACTCTGAATTTGCTC-3' (SEQ ID NO: 2) are used (the underlined parts are restriction enzyme sequences). Both termini of the PCR product are digested with restriction enzymes BamHI and HindIII and incorporated into glutathione-S-transferase (GST)-fused protein expression vector pGEX-2TH (Amersham Bioscience). The obtained GST-MCAF1 (1-334) expression vector is introduced into E. coli BL21 star (Stratagene), and selection is performed on an LB plate (10 g of NaCl, 10 g of tryptone, and 5 g of yeast extract per 1 liter; adjusted to pH7.0 with NaOH) containing ampicillin (50 µg/ml; hereinafter, referred to simply as "amp"). In 1.5 liters of LB/amp medium, E. coli expressing GST-MCAF1 (1-334) is cultured at 37°C. When the O.D. value of the E. coli concentration becomes about 1.0, the culturing temperature is lowered to 25°C, and isopropyl-thio-β-D-galactopyranoside (IPTG) is added such that the final concentration thereof is 0.2 mM. Then, the culturing is performed at 25°C for 20 hours. E. coli is recovered by centrifugation performed at 6,000 rpm for 5 minutes, dissolved in phosphate buffered saline (PBS) containing 1% Triton X-100, and disrupted with an ultrasonic disruption method. The insoluble fraction is precipitated by centrifugation performed at 1,200 rpm for 20 minutes, and the supernatant is transferred to a different vessel. Glutathione agarose beads (200 mg) are added to the supernatant and then shaken at 4°C for 1 hour. The beads are washed with PBS containing 1% Triton X-100, and then washed only with PBS to remove impurities and the like. Proteins finally bound to the beads are caused to flow out from the beads by adding 5 mM glutathione (GSH). The resultant recombinant protein solution is put into a semipermeable membrane tube and dialyzed against PBS.

### (2) Production of a polyclonal antibody against MCAF1

A solution containing GST-fused MCAF1 protein (1 mg) is added to an equivalent amount of Freund's adjuvant complete and thoroughly mixed. Then, the resultant mixture is inoculated into a rabbit (12 weeks of age) intradermally. Every 3 weeks, a solution containing the same recombinant protein (0.5 mg) is added to an equivalent amount of Freund's adjuvant incomplete, and the resultant mixture is inoculated into the rabbit in the same manner to immunize the rabbit repeatedly. After the rabbit is inoculated for amplification 4 times, antiserum of the rabbit is sampled.

### (3) Purification of the polyclonal antibody against MCAF1

For affinity purification of the antibody, the same amino group terminus region of MCAF1 as above (amino acid number: 1-334) is incorporated into His-fused protein expression vector pRSET (Invitrogen). The expression vector is introduced into E. coli BL21 star, and culturing is performed in 1.5 liters of LB/amp medium at 37°C. When the O.D. value of the E. coli concentration becomes about 1.0, the culturing temperature is lowered to 25°C, and IPTG is added such that the final concentration thereof is 0.2 mM. Then, the culturing is performed at 25°C for 20 hours. E. coli is recovered by centrifugation performed at 6,000 rpm for 5 minutes, dissolved in PBS containing 1% Triton X-100, and disrupted with an ultrasonic disruption method. The insoluble fraction is precipitated by centrifugation performed at 1,200 rpm for 20 minutes, and the supernatant is transferred to a different vessel. Nickel beads in gel volume (2 ml) are added to the supernatant and then shaken at 4°C for 1 hour. The beads are washed with PBS containing 1% Triton X-100, and then washed only with PBS to remove impurities and the like. Protein finally bound to the beads is caused to flow out from the beads with 300 mM imidazole and dialyzed against PBS. His-fused MCAF1 (1-334) protein (1 mg) for antibody purification is mixed with 200 ml ofAffigel 15 (BioRad) to immobilize the protein on the beads. After the extra binding group is saturated with an ethanol amine solution (1 M), the protein is mixed with rabbit antiserum and shaken at 4°C for about 12 hours. The beads are washed with an antibody washing buffer (1 M urea, 1 M NaCl, 20 mM Hepes (pH7.4)) and then eluted with an elution buffer (0.1 M glycine-HCl (pH2.5), 10% ethyleneglycol). Immediately thereafter, the eluted substance is neutralized with 1/20 volume of 1 M Tris-HCl (pH9.0). Then, the antibody solution is dialyzed against PBS. Thus, a specific antibody recognizing the amino group terminus region of MCAF1 is obtained.

### Example 2: Production of recombinant protein of MCAF2 and a polyclonal antibody against MCAF2

### (1) Synthesis of recombinant protein of MCAF2

The gene of the complete length of human MCAF2 (amino acid number: 1-681) is amplified by PCR using the following synthetic oligonucleotide primers. As the template, a vector of pcDNA3-MCAF2 (Ichimura T, Watanabe S, Sakamoto Y, Aoto T, Fujita N, and Nakao M., Transcriptional repression and heterochromatin formation by MBD1 and MCAF/AM family proteins, J. Biol. Chem. 280: 13928-13935, 2005) is used. The amplification is performed by repeating 35 times the cycle of reaction at 94°C for 1 minute, 60°C for 1 minute, and 72°C for 2 minutes. As the primer sequences, the sense strand of
5'-CGGGATCCCTCGAGATGGCAAGTCCAGATAGAAGTA -3' (SEQ ID NO: 3) and the anti-sense strand of
5'-ATTGGTACCCTCGAGTTACGTAAGATTTTCAGAAAACC -3' (SEQ ID NO: 4) are used (the underlined parts are restriction enzyme sequences). Both termini of the PCR product are digested with restriction enzyme Xhol and incorporated into GST-fused protein expression vector pGEX-4T1. The obtained GST-MCAF2 (1-681) expression vector is introduced into E. coli BL21 star, and culturing is performed in 1.5 liters of LB/amp medium at 37°C. When the O.D. value of the E. coli concentration becomes about 1.0, the culturing temperature is lowered to 25°C, and isopropyl-thio-β-D-galactopyranoside (IPTG) is added such that the final concentration thereof is 0.2 mM. Then, the culturing is performed at 25°C for 20 hours. E. coli is recovered by centrifugation performed at 6,000 rpm for 5 minutes, dissolved in phosphate buffered saline (PBS) containing 1% Triton X-100, and disrupted with an ultrasonic disruption method. The insoluble fraction is precipitated by centrifugation performed at 1,200 rpm for 20 minutes, and the supernatant is transferred to a different vessel. Glutathione agarose beads (200 mg) are added to the supernatant and then shaken at 4°C for 1 hour. The beads are washed with PBS containing 1% Triton X-100, and then washed only with PBS to remove impurities and the like. The procedure so far is the same as that for MCAF1. Since the flow efficiency of the complete length protein of MCAF2 from the beads with GSH is very low, the beads themselves bound to the protein are made into SDS with a sample buffer (2% SDS, 10% glycerol, 100 mM DTT, 60 mM Tris (pH6.8), 0.002% bromo phenol blue) and subjected to electrophoresis with 8% acrylic amide gel. Thus, a band of GST-fused MCAF2 protein classified as having a molecular weight of about 125 kDa is taken out from the gel.

### (2) Production and purification of a polyclonal antibody against MCAF2

A gel portion containing GST-fused MCAF2 protein (about 1 mg) is homogenized thoroughly, and is inoculated into a rabbit intradermally. An equivalent amount of gel portion as that of the initial inoculation is inoculated into the rabbit every 3 weeks. After the rabbit is inoculated 5 times in total, serum is recovered. For affinity purification of the polyclonal antibody, an amino group terminus region of MCAF2 (amino acid number: 1-328) is taken out with restriction enzymes BamHI and Bg1II and incorporated into His-fused protein expression vector pET28a (Novagen). The vector is introduced into E. coli BL21star, and soluble MCAF2 protein is synthesized in an LB/amp medium (1.5 liters). Affinity purification is performed by substantially the same procedure as for MCAF1. Thus, a specific antibody recognizing the amino group terminus region of MCAF2 is obtained.

### Example 3: Immunostaining on MCAF1 in normal lung tissue and different histologic types of lung cancers

A slide glass plate having paraffin tissue pieces (normal lung tissue and different histologic types of lung cancers) thereon is deparaffinated with xylene (5 minutes, 3 times) and then immersed in 100%, 90%, 80% and 70% ethanol sequentially for hydrophilation. Then, the resultant glass plate is washed with water. For activating the antigen, the pieces are heat-treated by a microwave oven (Nisshin EM Corporation; MWF-2 type) in 100 mM citric acid buffer solution (pH6.0) at 95°C for 15 minutes (this treatment is not necessary when staining is performed where the concentration of the primary antibody is twice or three times higher). The resultant substance is left until the liquid temperature becomes 60°C, and then washed with water. For the purpose of deactivating endogenous peroxidase, the pieces are immersed in 1/100 volume of methanol containing 30% hydrogen hydroxide solution to allow reaction at room temperature for 15 minutes. The resultant tissue pieces are washed with water again, then dried with air, and surrounded by PapPen (Daido Sangyo K.K.) on the slide glass plate. The pieces are washed with a washing buffer (50 mM Tris-HCl (pH7.3) and 0.1% Tween 20) for 15 minutes. The resultant pieces are blocked with an aqueous solution of blocking reagent (PerkinElmer) at room temperature for 5 minutes. Then, a primary antibody (rabbit anti-MCAF1 antibody; diluted 300 folds or 150 folds with antibody diluent (DAKO)) is added to allow reaction in a humidifier box at room temperature for 1 hour. After the resultant pieces are washed with the washing buffer 3 times each for 5 minutes, an envision plus anti-rabbit secondary antibody (DAKO) is added to allow reaction at room temperature for 45 minutes. The pieces are washed with the washing buffer 3 times each for 5 minutes, and a DAB solution (DAKO DAB substrate chromagen system) is added. After color development is kept for about 4 minutes, the reaction is stopped by washing the pieces with water. Then, rabbit IgG (Sigma) is used as a negative control, instead of the primary antibody, to carry out staining reaction in substantially the same manner. Then, the pieces are stained with hematoxylin as nuclear staining, dewatered with 70%, 80%, 90% and 100% ethanol, clarified with xylene, encapsulated with a glass cover, and subjected to observation with an optical microscope.

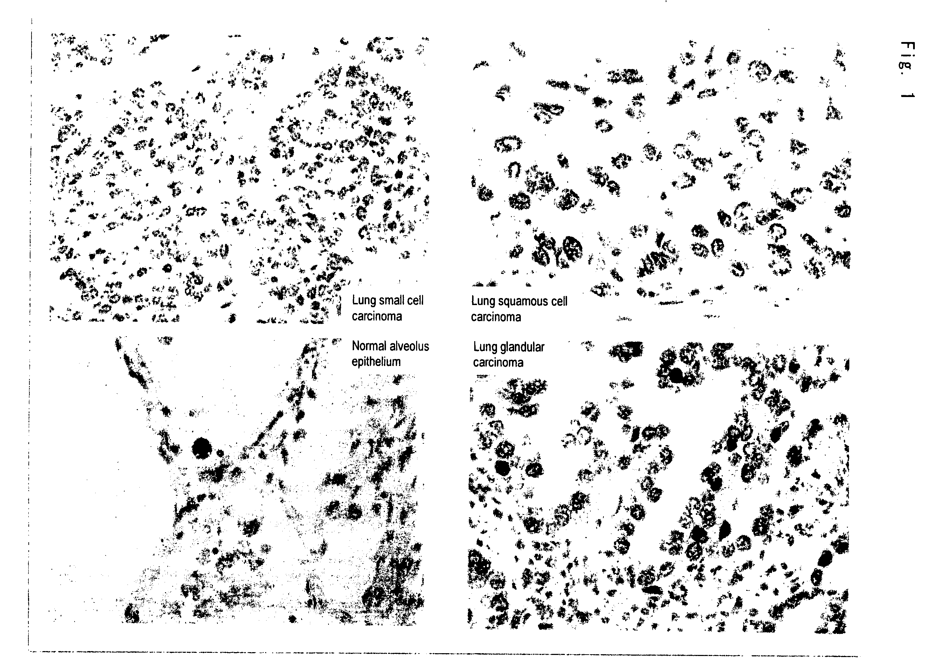

The results are shown in Fig. 1. In normal alveolus epithelial tissue, substantially no stained MCAF1 was observed. In the cancer cells of the different histologic types of lung cancer tissues (small cell carcinoma, squamous cell carcinoma, glandular carcinoma), MCAF1 was expressed significantly highly. Normal interstitial cells in the cancer tissues (fibroblast, etc.) were not stained. The above results were obtained by tissue immunostaining and hematoxylin eosin staining on MCAF1.

### Example 4: Immunostaining on MCAF1 in cervical cancer, uterine body cancer, gastric cancer and breast cancer

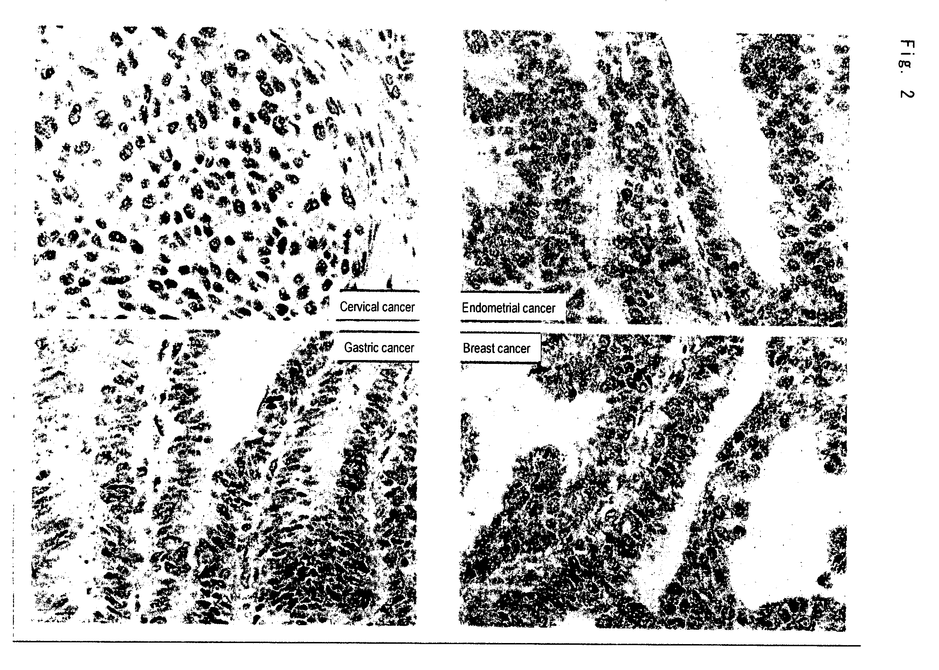

MCAF1 was immunostained in different histologic types of cancer tissues (cervical cancer, uterine body cancer, gastric cancer and breast cancer) in substantially the same manner as in Example 3. The results are shown in Fig. 2. In the cancer cells of the different histologic types of cancer tissues (cervical cancer, uterine body cancer, gastric cancer and breast cancer), MCAF1 was expressed significantly highly. Normal interstitial cells in the cancer tissues (fibroblast, etc.) were not stained. The above results were obtained by tissue immunostaining and hematoxylin eosin staining on MCAF1.

### Example 5: Immunostaining on MCAF1 in precancerous lesion (severe dysplasia), early stage (initial invasive cancer), and lymph node metastasis of cervical cancer

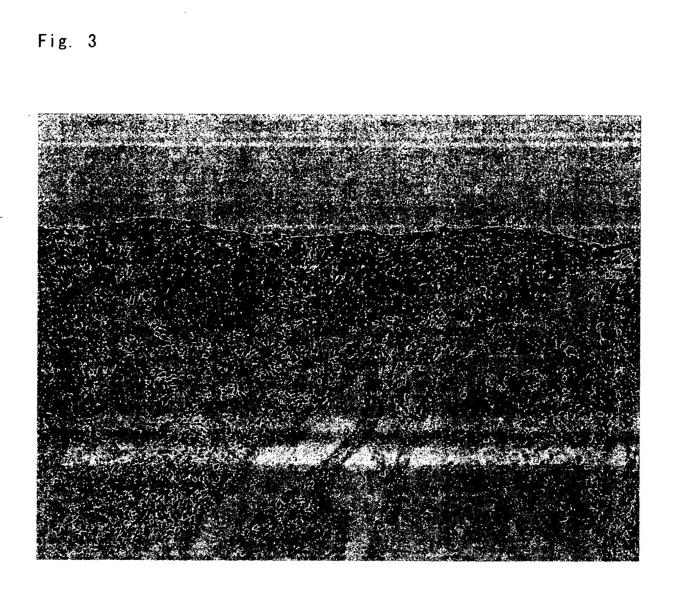

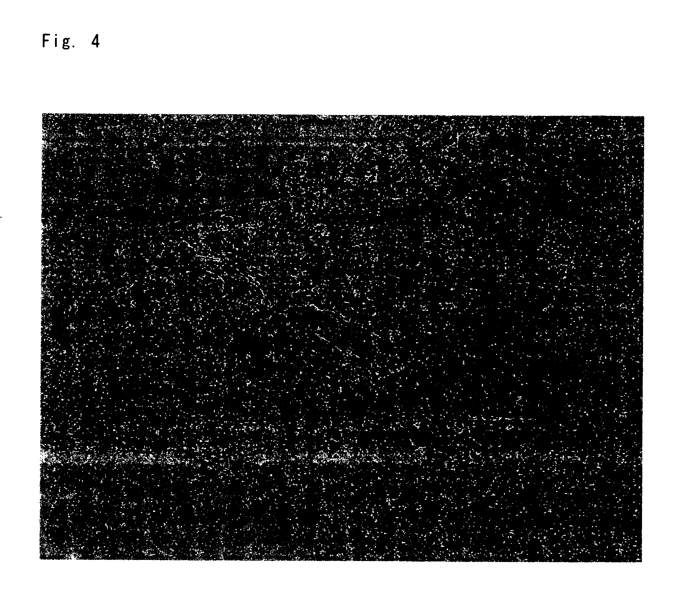

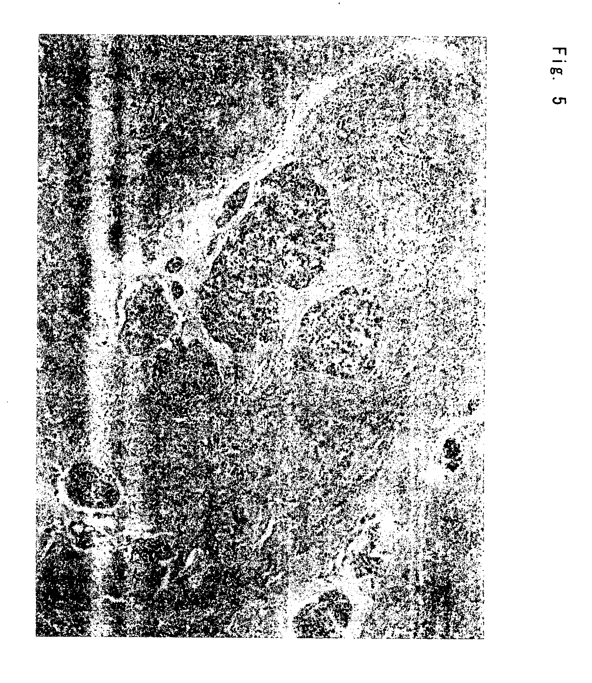

MCAF1 was immunostained in precancerous lesion (severe dysplasia) samples (paraffin pieces), early-stage (initial invasive cancer) samples and lymph node metastasis samples derived from cervical cancer patients in substantially the same manner as in Example 3. The results are shown in Fig. 3, Fig 4 and Fig 5 respectively. As shown here, in the precancerous lesion sample, early-stage sample and lymph node metastasis sample derived from the cervical cancer patient, MCAF1 exhibited strong stainability. These results indicate that MCAF1 is highly expressed in these tissues.

### INDUSTRIAL APPLICABILITY

A method, an agent and a kit for diagnosing tumor according to the present invention are useful for diagnosing a wide range of histologic types of tumors. The method, the agent and the kit for diagnosing tumor according to the present invention are also usable for diagnosing precancerous lesion, initial invasive cancer, and lymph node metastasis of cervical cancer, and therefore are especially useful for early-stage treatment and establishment of an accurate treatment principle of cervical cancer.

## Claims

1. A diagnostic agent for tumor, comprising an antibody against MCAF 1 or MCAF2.

2. A diagnostic agent for tumor, comprising F(ab')₂ fragment or Fab' fragment of an antibody against MCAF1 or MCAF2.

3. The diagnostic agent according to claim 1 or 2, wherein the tumor is lung cancer, cervical cancer, gastric cancer or breast cancer.

4. The diagnostic agent according to claim 3, wherein the tumor is cervical cancer.

5. A diagnostic method for tumor, comprising the steps of:
putting a sample derived from a subject into contact with an antibody against MCAF 1 or MCAF2; and
detecting or measuring the MCAF1 or MCAF2 in the sample.

6. The diagnostic method according to claim 5, wherein F(ab')₂ fragment or Fab' fragment of the antibody against the MCAF1 or MCAF2 is used as the antibody against the MCAF1 or MCAF2.

7. The diagnostic method according to claim 5 or 6, which is performed in accordance with enzyme immunoassay or radio immunoassay.

8. The diagnostic method according to any one of claims 5 through 7, wherein the tumor is lung cancer, cervical cancer, gastric cancer or breast cancer.

9. The diagnostic method according to claim 8, wherein the tumor is cervical cancer.

10. The diagnostic method according to any one of claims 5 through 9, wherein the sample is tissue, cell, serum, saliva or urine derived from the subject.

11. A diagnostic kit for tumor, comprising an antibody against MCAF1 or MCAF2.

12. A diagnostic kit for tumor, comprising F(ab')₂ fragment or Fab' fragment of an antibody against MCAF1 or MCAF2.

13. The diagnostic kit according to claim 11 or 12, wherein the tumor is lung cancer, cervical cancer, gastric cancer or breast cancer.

14. The diagnostic kit according to claim 13, wherein the tumor is cervical cancer.

15. A diagnostic agent for precancerous lesion, comprising an antibody against MCAF1 or MCAF2.

16. The diagnostic agent according to claim 15, wherein the precancerous lesion is precancerous lesion of cervix.

17. A diagnostic kit for precancerous lesion, comprising an antibody against MCAF1 or MCAF2.

18. The diagnostic kit according to claim 17, wherein the precancerous lesion is precancerous lesion of cervix.

19. A diagnostic method for precancerous lesion, comprising the steps of:
putting a sample derived from a subject into contact with an antibody against MCAF1 or MCAF2; and
detecting or measuring the MCAF1 or MCAF2 in the sample.

20. A diagnostic method for precancerous lesion of cervical cancer, comprising the steps of:
putting a sample derived from cervix or a tissue in the vicinity thereof of a subject into contact with an antibody against MCAF1 or MCAF2; and
detecting or measuring the MCAF1 or MCAF2 in the sample.

21. A diagnostic agent for lymph node metastasis, comprising an antibody against MCAF1 or MCAF2.

22. The diagnostic agent according to claim 21, wherein the lymph node metastasis is lymph node metastasis of cervical cancer.

23. A diagnostic kit for lymph node metastasis, comprising an antibody against MCAF1 or MCAF2.

24. The diagnostic kit according to claim 23, wherein the lymph node metastasis is lymph node metastasis of cervical cancer.

25. A diagnostic method for lymph node metastasis, comprising the steps of:
putting a sample derived from lymph node of a subject into contact with an antibody against MCAF 1 or MCAF2; and
detecting or measuring the MCAF 1 or MCAF2 in the sample.

26. The diagnostic method according to claim 25, which is for diagnosing lymph node metastasis of cervical cancer.
